# EUROPÄISCHE PATENTSCHRIFT

(11) **EP 2 651 471 B1**
(45) Veröffentlichungstag und Bekanntmachung des Hinweises auf die Patenterteilung: **27.07.2016**
(21) Anmeldenummer: 11799667.8
(22) Anmeldetag: 14.12.2011
(51) Int. Cl.: A61M 5/162

(54) **VORRICHTUNG FÜR DIE ENTNAHME EINER FLÜSSIGKEIT AUS EINEM BEHÄLTER**
DEVICE FOR WITHDRAWING LIQUID FROM A CONTAINER
DISPOSITIF PERMETTANT DE PRÉLEVER DU LIQUIDE CONTENU DANS UN RÉCIPIENT

(30) Priorität: 17.12.2010 EP 10195706
(43) Veröffentlichungstag der Anmeldung: 23.10.2013
(73) Patentinhaber: Weibel CDS AG, 9104 Waldstatt (CH)
(72) Erfinder: WEIBEL, Ludwig Daniel, CH-9104 Waldstatt (CH)
(74) Vertreter: Hepp Wenger Ryffel AG
(86) Internationale Anmeldenummer: PCT/EP2011/072716
(87) Internationale Veröffentlichungsnummer: WO 2012/080310

(56) Entgegenhaltungen:
- WO-A1-01/00261
- DE-A1- 2 647 624
- FR-A1- 2 753 624

## Beschreibung

Die Erfindung betrifft eine Vorrichtung für die Entnahme einer Flüssigkeit aus einem Behälter gemäss dem Oberbegriff von Anspruch 1. Dabei handelt es sich in der Regel um Vorrichtungen für die nadellose Entnahme von pharmazeutischen Substanzen aus einem hermetisch verschlossenen Glasgefäss. Die Flüssigkeit kann dabei in eine Injektionsspritze überführt werden, wobei die Nadel erst nachträglich aufgesetzt wird. Selbstverständlich können derartige Vorrichtungen aber auch im nichtmedizinischen Bereich eingesetzt werden, beispielsweise für chemische Analysezwecke im Lebensmittelbereich usw.

Durch die WO 01/60436 ist eine gattungsmässig vergleichbare Vorrichtung bekannt geworden, bei der eine linear verschiebbare Aufstechspitze in einem rohrförmigen Gehäuse gehalten ist, das auf die geschlossene Behältermündung aufgesetzt ist. Die Aufstechspitze wird in der oberen Ruhelage durch flexible Rastnocken fixiert, und sie weist eine umlaufende Verzahnung auf, welche in korrespondierende seitliche Zahnsegmente eingreifen. Bei einem Druck von oben in Richtung Behälter nach dem Anschluss einer Spritze löst sich die Aufstechspitze aus der Verrastung und gleitet in der Verzahnung drehfest nach unten, bis der Verschlussstopfen auf dem Behälter durchstochen ist. Anschliessend kann mit der Injektionsspritze der Behälterinhalt angesaugt werden.

Durch die FR 2 753 624 ist eine Verbindungsvorrichtung zwischen einem ersten und einem zweiten Gefäss bekannt geworden, die eine Perforationsvorrichtung zum Durchdringen eines Deckels am ersten Gefäss aufweist. Die Perforationsvorrichtung wird an einem Kolben geführt, der auf der Innenseite eines rohrförmigen Aufsatzes verschiebbar ist. Durch Druck auf die Perforationsvorrichtung gleitet diese nach unten und verrastet sich in der Entnahmestellung.

Ein erheblicher Nachteil bekannter Vorrichtungen besteht darin, dass auch nach dem Durchstössen des Verschlusselements mit der Hohlnadel das Innere des Behälters gegenüber der Atmosphäre abgedichtet ist, wenn an das Penetrationselement ein anderer Behälter wie z.B. eine Injektionsspritze angeschlossen wird. Beim Ansaugen der Flüssigkeit aus dem Behälter entsteht somit ein Unterdruck, der im Extremfall dazu führt, dass der grösste Teil der Flüssigkeit wieder in den Behälter zurückströmt, sobald die Pumpbewegung unterbrochen wird. Aufgrund der Flexibilität des Verschlusselements kann zwar bei starkem Unterdruck im Behälter allenfalls Luft entlang der Aussenseite der Hohlnadel nachströmen. Dies ist allerdings nicht erwünscht, weil dadurch mikrobiologische Verunreinigungen in den Behälter und damit in die Flüssigkeit gelangen können.

Es ist daher eine Aufgabe der Erfindung, eine Vorrichtung der eingangs genannten Art zu schaffen, bei der die erwähnten Nachteile vermieden werden und bei der insbesondere unter Aufrechterhalten von sterilen Bedingungen ein Rückströmen von Flüssigkeit in den Behälter vermieden wird. Die Vorrichtung soll ausserdem zuverlässig funktionieren und auch von nicht besonders geschultem Personal leicht zu bedienen sein. Ausserdem soll sie einfach und preiswert herzustellen sein, da die Vorrichtung Bestandteil einer Einwegverpackung ist, die nach Gebrauch entsorgt werden muss.

Diese Aufgaben werden erfindungsgemäss mit einer Vorrichtung gelöst, welche die Merkmale im Anspruch 1 aufweist. Der wenigstens eine vorzugsweise entlang der Hohlnadel verlaufende Belüftungskanal bewirkt ersichtlicherweise, dass das Innere des Behälters in der Entnahmelage mit der Atmosphäre kurzgeschlossen wird. Der Aufbau eines stetig steigenden Unterdrucks im Behälter wird damit vermieden, da jeweils so viel Luft in den Behälter nachströmt, wie Flüssigkeit entfernt wird. Trotzdem ist eine schmutzfreie und sogar eine keimfreie Umgebung im Behälter gewährleistet, wenn gemäss einer weiteren Ausgestaltung die zugeführte Luft ein Filter passieren muss. Je nach Beschaffenheit dieses Filters können auch kleinste Partikel oder Lebewesen zurückgehalten werden. Selbstverständlich ist es denkbar, mehrere Lüftungskanäle anzuordnen und diese können entlang der Hohlnadel entweder linear oder auch schraubenlinienförmig verlaufen. Denkbar wäre auch die Anordnung eines Belüftungskanals an einem von der Hohlnadel getrennten Element, beispielsweise in der Form einer separaten Belüftungsnadel.

Besonders vorteilhaft ist jedoch der Belüftungskanal als Nut auf der Aussenseite der Hohlnadel ausgebildet. Vorzugsweise sind es mehrere parallele Nuten. Ein derartiges Bauteil ist einfach herzustellen und bei ausreichender Nuttiefe wird selbst dann ein genügend grosser Durchtrittsquerschnitt freigehalten, wenn das Verschlusselement aus sehr weichem und elastischem Material besteht.

Das Filter kann grundsätzlich am Penetrationselement angeordnet sein und sich zusammen mit diesem verschieben, oder es kann starr am Aufsatz gehalten sein. Wichtig ist in jedem Fall, dass die dem Belüftungskanal zugewandte Seite des Filters Bestandteil eines gegenüber der Atmosphäre steril abgegrenzten Raumes ist. Das Filter kann beispielsweise am Penetrationselement vorzugsweise am atmosphärenseitigen Ende des Belüftungskanals angeordnet sein, und zwar beispielsweise an einem die Hohlnadel umgebenden Kragen, der mit Öffnungen versehen ist und der gleichzeitig das Filter abstützt bzw. trägt. Diese nahe am Belüftungskanal angeordnete Variante hat den Vorteil, dass das Filter flächenmässig relativ klein gehalten werden kann.

Bei einer Zuordnung des Filters am Aufsatz erfolgt die Anordnung vorzugsweise in einem dem Verschlusselement zugewandten Bereich. Das Filter kann dabei in einer Halterung angeordnet sein, welche das Penetrationselement kreisringförmig umgibt. Diese Anordnung hat den Vorteil, dass der in der Ruhelage steril zu haltende Raum sehr klein gehalten werden kann.

Erfindungsgemäss weisen die Führungsmittel wenigstens einen Wandabschnitt auf, der das Penetrationselement mit dem Aufsatz verbindet und dieses in der Ruhelage hält, wobei der Wandabschnitt derart verformbar ist, dass er das Penetrationselement bis zum Erreichen der Entnahmelage führt. Durch die feste Verbindung zwischen Aufsatz und Penetrationselelement ist einerseits eine Verdrehsicherung gewährleistet, anderseits in Folge der Verformbarkeit aber auch eine gute Geradeführung. Eine derartige Ausgestaltung der Führungsmittel wäre auch an konventionellen Vorrichtungen ohne Entlüftungskanal und ohne Filter sehr vorteilhaft.

Der verformbare Wandabschnitt ist wenigstens eine das Penetrationselement umgebende, federelastische Membran. Diese kann derart verformbar sein, dass sie das Penetrationselement in der Entnahmelage unter der Federvorspannung fixiert. Die kuppelartig ausgebildete Membran ist somit in Folge ihrer Federeigenschaften sowohl in der Ruhelage als auch in der Entnahmelage vorgespannt. Dabei wird der "Knackfrosch"-Effekt der Membran ausgenutzt. Selbstverständlich können zum Fixieren des Penetrationselements in der Entnahmelage aber auch Rastmittel angeordnet sein, so dass die Federvorspannung der Membran für das Halten der Entnahmelage unerheblich ist.

Die Membran kann ganz unterschiedlich ausgebildet sein und beispielsweise auch in Umfangsrichtung aus mehreren, von einander abgetrennten Segmenten bestehen. Über die Zwischenräume zwischen den Segmenten kann in der Entnahmelage Atmosphärenluft nachströmen. Es ist aber auch denkbar, dass eine in der Ruhelage geschlossene Membran mit Sollbruchstellen versehen ist, welche beim Überführen des Penetrationselements in die Entnahmelage aufplatzen. Derartige Sollbruchstellen haben den Vorteil, dass die Membran in der Ruhelage relativ formsteif ist und erst nach dem Aufplatzen der Sollreisslinien eine erhöhte Elastizität aufweist. Ausserdem kann auch hier in der Entnahmelage über die aufgeplatzten Sollreisslinien Luft aus der Atmosphäre nachströmen.

Ein wesentlicher Vorteil wird in jedem Fall erreicht, wenn die Führungsmittel einstückig mit dem Aufsatz und/oder mit dem Penetrationselement verbunden sind. Die gesamte Struktur lässt sich auf diese Weise als Spritzgussteil in Kunststoffmaterial einstückig herstellen. In bestimmten Fällen kann es jedoch fabrikationstechnisch auch zweckmässig sein, einzelne Bauteile separat herzustellen und anschliessend miteinander zu verschweissen oder auf andere Weise miteinander zu verbinden.

Beispielsweise können in einer gegebenenfalls ebenfalls zu bevorzugenden Ausführung die Führungsmittel Befestigungsmittel aufweisen, welche zum Verbinden der Führungsmittel mit dem Aufsatz an diesem verrastbar sind. Vorzugsweise erfolgt die Verrastung dabei derart, dass die beiden Teile unlösbar, d.h. nicht zum Lösen durch einen Benutzer vorgesehen, miteinander verbunden sind. Beispielsweise können die Befestigungsmittel Rasthaken aufweisen, welche in entsprechende Rastausnehmungen am Aufsatz eingreifen und dort verrasten. Dabei können die Führungsmittel z.B. einstückig mit dem Penetrationselement oder über weitere Befestigungsmittel mit diesem verbunden sein. Selbstverständlich sind je nach Erfordernis auch andere Verbindungsarten denkbar bei welchen die Führungsmittel z.B. mittels Laser- oder Ultraschallschweissen oder aber auch durch eine Verklebung mit dem Aufsatz verbunden sind.

Damit kann eine Ausführungsform des Penetrationselements bei unterschiedlich ausgebildeten Aufsätzen zur Anwendung kommen. Beispielsweise können verschiedene Aufsätze an unterschiedliche Behälter angepasst sein, während identisch ausgebildete Penetrationselemente mit den genannten Befestigungsmitteln mit den Aufsätzen verbunden werden können. Zudem können auf diese Weise unterschiedliche Materialeigenschaften optimal miteinander gepaart werden. Optimal sind dabei Werkstoffe, welche mit verschiedenen Verfahren sterilisierbar sind (z. B. strahlensterilisierbar, dampfsterilisierbar), ohne dabei Schaden zu erleiden.

Mit Vorteil kann der Aufsatz auch zweiteilig oder mehrteilig ausgebildet sein, wobei ein erster Teil zum Ankoppeln an den Behälter vorgesehen ist und ein zweiter Teil mit dem Penetrationselement verbunden ist. Hieraus ergibt sich der Vorteil, dass beispielsweise eine Ausführung des zweiten Teils für unterschiedliche Ausbildungen des ersten Teils, z.B. für unterschiedliche Behälter ausgebildet, genutzt werden kann. Die wenigstens zwei Teile sind bevorzugt über Verrastungen oder z.B. über eine Verschweissung mittels Laser oder Ultraschall oder eine Klebung miteinander verbunden. Grundsätzlich können aber auch einstückige Ausführungen der Aufsätze vorteilhaft sein.

Um einen keimfreien und möglichst luft- bzw. flüssigkeitsdichten Anschluss an das Penetrationselement zu gewährleisten, ist dieses mit einem entsprechenden Kupplungsteil versehen. Dabei kann es sich beispielsweise um den Innenkegel eines Luer-Locks handeln. Aber auch andersartige Kupplungsmittel wie z.B. beliebige Schnappverschlüsse oder dergleichen sind denkbar.

Das Penetrationselement kann ausserdem Rastmittel aufweisen, mit denen es in der Entnahmelage verrastbar ist. Dadurch ist gewährleistet, dass bei der Entnahme der Flüssigkeit keine unerwünschten Relativbewegungen auftreten.

Bevorzugt sind diese Rastmittel derart am Penetrationselement angeordnet, dass sie zur Manipulation durch einen Benutzer von aussen zugänglich sind. Damit kann gegebenenfalls eine Verrastung manuell hergestellt werden, sollte diese z.B. nicht ordnungsgemäss automatisch erfolgen. Insbesondere können die Rastmittel ausserhalb des beispielsweise als Membran ausgebildeten verformbaren Wandabschnitts angeordnet sein. Vorzugsweise weist das Penetrationselement eine Fingerplatte auf, welche zum Verschieben zwischen Ruhelage und Entnahmelage vorgesehen ist. Die Fingerplatte bildet dabei ein Widerlager für die Finger eines Benutzers, um auf einfache Weise den Übergang von der Ruhelage in die Entnahmelage durchführen zu können. Mit Vorteil können die Rastmittel an der Fingerplatte ausgebildet sein, z.B. als sich zum Aufsatz hin erstreckende Rasthaken oder Rastzungen.

Bevorzugt weist der Aufsatz in diesem Fall Rastmittel auf, welche zu den Rastmitteln des Penetrationselements komplementär sind, sodass das Penetrationselement mit seinen Rastmitteln am Aufsatz verrastbar ist. In der Entnahmelage bilden Aufsatz und Penetrationselement somit eine gut fixierte und robuste Anordnung. Es versteht sich, dass das Penetrationselement in der Entnahmelage in Varianten auch anderswo verrastet sein kann, beispielsweise direkt am Bördelrand eines angeschlossenen Behälters. Eine Verrastung lässt sich auf diese Weise allerdings weniger gut kontrollieren, da sie nicht innerhalb der Vorrichtung alleine erfolgt.

Der Aufsatz kann einen Zentrierring tragen, der die Hohlnadel zusätzlich führt und zentriert. Das Durchstechen des Verschlusselements erfolgt so an einer genau definierten Stelle. Ausserdem werden unerwünschte Querbewegungen durch fehlerhafte Handhabung beim Einstechen vermieden.

Zum Schutz des Penetrationselements in der Ruhelage und auch als Garantiesicherung ist das Penetrationselement durch eine lösbare Schutzkappe abgedeckt. Diese Schutzkappe muss ausreichend Stabilität aufweisen, um auch bei starken Schlägen und Stössen eine Verschiebung des Penetrationselements zu verhindern. Als Garantiesicherung kann die Schutzkappe beispielsweise ein abtrennbares Garantieband aufweisen. Die Schutzkappe hat zusätzlich auch noch die Aufgabe die Vorrichtung keimfrei abzudichten. Dies setzt voraus, dass die Schutzkappe dichtend gegen das Verschlusselement und/oder gegen die Behältermündung gepresst wird. Dies kann beispielsweise über eine Manschette erfolgen, welche um den Aufsatz und um die Behältermündung aufgeschrumpft oder angerollt oder angebördelt wird. Bei einer Ausbildung dieser Manschette beispielsweise aus Aluminium oder aus einer Aluminiumlegierung kann das eine Ende der Manschette bei der Herstellung direkt in den Aufsatz eingegossen werden. Nach dem Füllen und Verschliessen des Behälters kann das freie Ende um die Behältermündung angerollt oder angebördelt werden.

Die Schutzkappe kann besonders vorteilhaft auch mit einem Gewinde versehen und auf den Aufsatz und/oder auf das Penetrationselement aufgeschraubt sein. Eine Fixierung der Schraubkappe könnte beispielsweise über eine Schweissung erfolgen.

Eine sehr vorteilhafte Möglichkeit der Befestigung auf einer Behältermündung besteht auch darin, dass der Aufsatz auf der Behältermündung aufgeschnappt ist. Bei entsprechend ausgebildeten Rastmitteln kann dabei eine relativ hohe Anpresskraft aufgebaut werden. Vergleichbare Schnappverbindungen auf Behältermündungen sind beispielsweise bereits bei Verschlüssen in der Lebensmittelindustrie bekannt.

Eine weitere sehr vorteilhafte Ausführung besteht in der Wahl von möglichst transparenten Kunststoffen für die einzelnen Bauteile. Dadurch kann das fertig abgefüllte und montierte Produkt zerstörungsfrei visuell auf jegliche Defekte inspiziert werden.

Es hat sich gezeigt, dass ein Kunststoff besonders vorteilhaft ist, welcher einen gewissen Anteil von Glaskugeln oder eines Glasgranulats aufweist. Es hat sich gezeigt, dass ein Anteil von 5% - 20%, bevorzugt 10% - 15% besonders gute Eigenschaften hinsichtlich der Stabilität, insbesondere in der Ruhelage, sowie des Bruch- bzw. Reissverhaltens der Membran beim Übergang in die Entnahmelage E ergibt.

Weitere Vorteile und Einzelmerkmale der Erfindung sind in den Zeichnungen dargestellt und werden nachstehend beschrieben. Es zeigen:
- Figur 1:: Einen stark vergrösserten Querschnitt durch eine erfindungsgemässe Vorrichtung in Ruhelage,
- Figur 2:: die Vorrichtung gemäss Figur 1 in Entnahmelage mit angesetzter Spritze,
- Figur 3:: einen Schnitt durch die Ebene I-I gemäss Figur 1,
- Figur 4:: einen Schnitt durch die Ebene II-II gemäss Figur 1,
- Figur 5:: einen Querschnitt durch ein nicht erfindungsgemässes Ausführungsbeispiel einer Vorrichtung,
- Figur 6:: die Vorrichtung gemäss Figur 5 in Entnahmelage,
- Figur 7:: ein weiteres leicht abgewandeltes und nicht erfindungsgemässes Ausführungsbeispiel der Vorrichtung gemäss Figur 5,
- Figur 8:: die Vorrichtung gemäss Figur 7 in Entnahmelage,
- Figur 9:: einen Querschnitt durch eine erfindungsgemässe Vorrichtung mit integriertem Entnahmeventil,
- Figur 10:: einen Querschnitt durch ein weiteres Ausführungsbeispiel einer Vorrichtung,
- Figur 11:: eine äussere Schrägansicht eines weiteren Ausführungsbeispiels einer Vorrichtung in einer Ruhelage ohne Aufsatz,
- Figur 12:: eine weitere äussere Schrägansicht des Ausführungsbeispiels gemäss Fig. 11,
- Figur 13:: eine Ausschnittsansicht auf eine Hohlnadel des Ausführungsbeispiels gemäss Fig. 11,
- Figur 14:: eine äussere Seitenansicht des Ausführungsbeispiels gemäss Fig. 11,
- Figur 15:: eine äussere Seitenansicht eines Aufsatzes für die Vorrichtung der Fig. 11,
- Figur 16:: eine Draufsicht auf den Aufsatz gemäss Fig. 15,
- Figur 17:: einen Längsschnitt durch den Aufsatz gemäss Fig. 15,
- Figur 18:: einen weiteren Längsschnitt durch den Aufsatz gemäss Fig. 15,
- Figur 19:: einen Längsschnitt durch eine weitere Ausführungsform eines zweiteiligen Aufsatzes für eine erfindungsgemässe Vorrichtung,
- Figur 20:: eine äussere Schrägansicht eines Kopplungsteils des Aufsatzes gemäss Fig. 19.

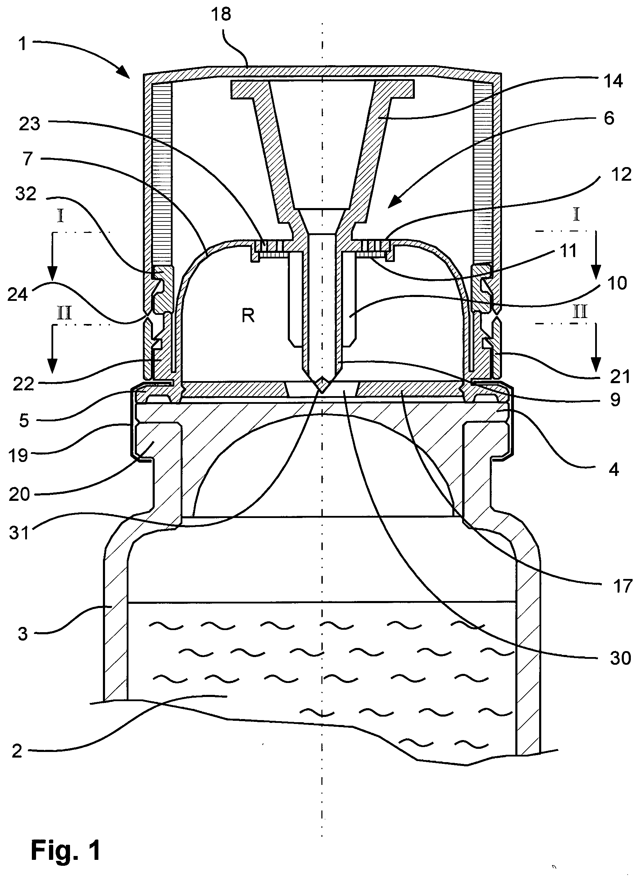

Figur 1 zeigt eine Anordnung bestehend aus einer erfindungsgemässen Vorrichtung 1, welche auf die Behältermündung 20 eines Behälters 3 aufgesetzt ist. Der Behälter 3, beispielsweise aus Glas gefertigt, ist mit einer Flüssigkeit 2 gefüllt und mit einem Verschlusselement 4, beispielsweise aus Gummi luftdicht verschlossen. Bei der Flüssigkeit 2 kann es sich um einen pharmazeutischen Wirkstoff handeln, der dazu bestimmt ist, mit einer Injektionsspritze entweder direkt in den menschlichen oder tierischen Körper injiziert zu werden oder aber einem Infusionssystem beigemischt zu werden.

Die Vorrichtung 1 besteht im Wesentlichen aus einem Aufsatz 5 aus Kunststoffmaterial, der mit seiner Unterseite dichtend auf dem äusseren Randbereich des Verschlusselements 4 aufliegt und der über eine kuppelartig gewölbte Membran 7 mit einem zentralen Penetrationselement 6 verbunden ist. Der Aufsatz 5 ist einstückig mit einem Wandabschnitt 22 verbunden, der als Widerlager für ein aussen liegendes Garantieband 21 dient. Die dichtende und formschlüssige Verbindung des Aufsatzes 5 mit der Behältermündung 20 erfolgt über eine Manschette 19 aus Aluminium, welche direkt in den Aufsatz 5 eingegossen ist und welche an ihrem freien Ende an die Behältermündung 20 angerollt ist. In einer Ebene unmittelbar über dem Verschlusselement 4 trägt der Aufsatz einen Zentrierring 17 mit einer zentralen Öffnung 30, der beispielsweise eingeschnappt werden kann.

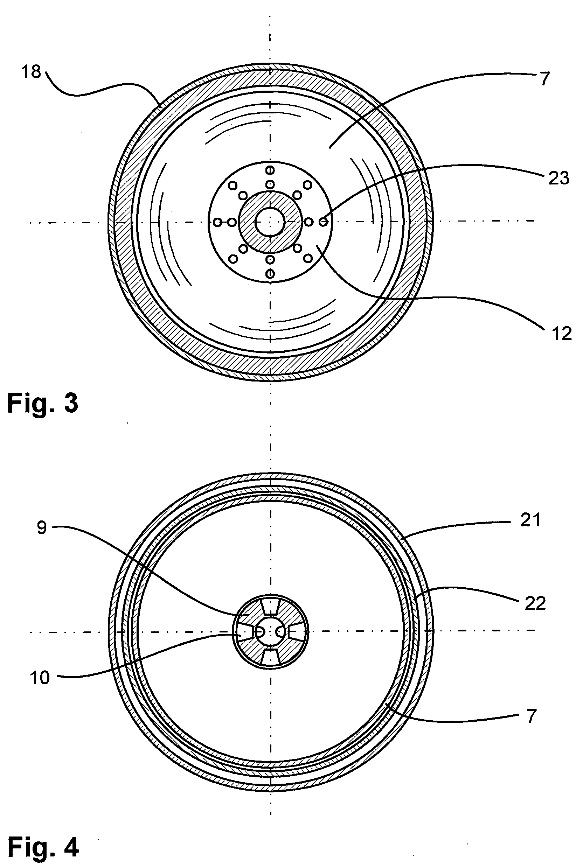

Das vorzugsweise rotationssymmetrisch ausgebildete Penetrationselement 6 verfügt im mittleren Abschnitt über einen umlaufenden Kragen 12, in welchem Ansaugöffnungen 23 angeordnet sind (Figur 3). Unterhalb des Kragens ist ein mikrobiologisch dichtes Filter 11 angeordnet, das sämtliche Ansaugöffnungen 23 überdeckt. Über dem Kragen 12 ist integral der Innenkonus eines Luer-Locks 14 angeordnet. Dieser geht unmittelbar in eine Hohlnadel 9 über, welche sich unterhalb des Kragens 12 nach unten erstreckt und welche in eine mit Öffnungen versehene Aufstechspitze 31 mündet. Der Begriff Hohlnadel wird hier für jedes Element verwendet, das einen Ansaugkanal bildet und das in der Lage ist, das Verschlusselement 4 zu penetrieren.

Wie insbesondere auch aus Figur 4 ersichtlich ist, hat die Hohlnadel 9 eine relativ dicke Wandung, entlang welcher vorzugsweise parallele Belüftungskanäle 10 in der Form von Nuten angeordnet sind. Diese Nuten erstrecken sich bis unmittelbar unter den Kragen 12. Das gesamte Penetrationselement 6 ist abgesehen vom Filter 11 vorzugsweise einstückig ausgebildet. Es kann in die Membran 7 eingeschweisst oder eingeschnappt sein oder es kann gar mit dieser ebenfalls einstückig ausgebildet sein. Die Belüftungskanäle können im Querschnitt auch als Schwalbenschwanznut ausgebildet sein. Damit kann die Gefahr reduziert werden, dass das elastische Material des Verschlusselements 4 die Belüftungskanäle verschliesst.

Die Membran 7 ist federelastisch ausgebildet, so dass sie in der dargestellten Ruhelage R das Penetrationselement 6 nach oben vorspannt. In dieser Ruhelage ist das Penetrationselement von einer Schutzkappe 18 abgedeckt, welche über eine Sollbruchlinie 24 mit dem Garantieband 21 verbunden ist. Ein Dichtring 32 zwischen dem Wandabschnitt 22 und der Schutzkappe 18 sorgt dafür, dass der gesamte Innenraum der Schutzkappe über dem Verschlusselement 4 steril und hermetisch abgedichtet ist. Die Schutzkappe 18 kann aus Kunststoffmaterial oder auch aus Aluminium gefertigt sein. In bestimmten Fällen wäre es denkbar, dass der Luer-Lock 14 in der Ruhelage R durch die Schutzkappe 18 gehalten bzw. zentriert wird.

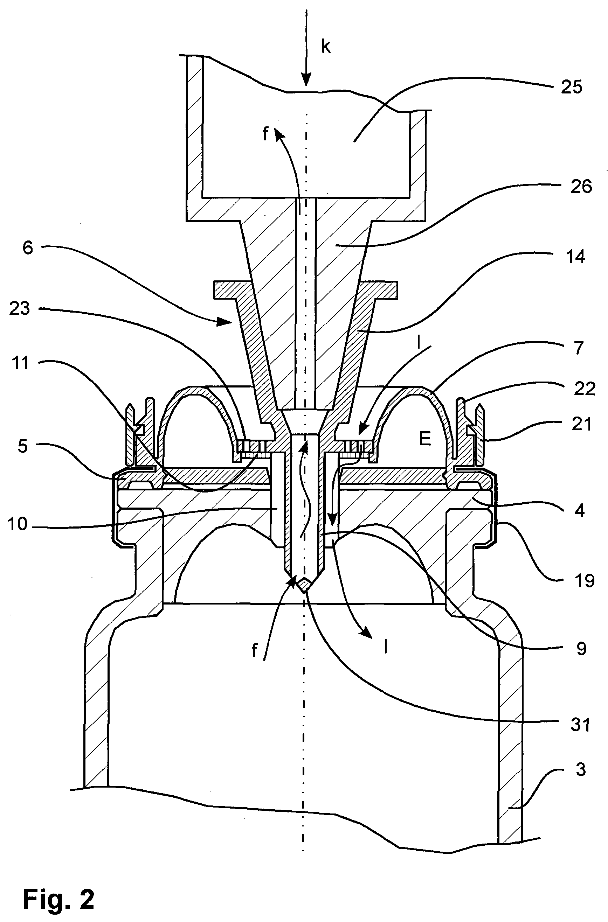

Anhand von Figur 2 wird nachstehend die Entnahme der Flüssigkeit aus dem Behälter 3 in der Entnahmelage E erläutert. Bevor die Entnahmelage erreicht wird, muss zuerst die Schutzkappe 18 entfernt werden, so dass am Aufsatz 5 nur noch das Garantieband 21 zurückbleibt. Je nach Garantiesystem könnte beim Öffnen der Schutzkappe auch das Garantieband wegfallen. Sofort nach dem Entfernen der Schutzkappe wird der Gegenkonus 26 einer Spritze 25 in den Luer-Lock 14 eingeführt und es wird eine Kraft in Pfeilrichtung k auf das Penetrationselement 6 ausgeübt. Dabei durchdringt die Hohlnadel 9 das Verschlusselement 4, so dass die Aufstechspitze 31 ins Innere des Behälters 3 hineinragt. Die Belüftungskanäle 10 schaffen dabei zusätzlich eine Verbindung zwischen dem Innenraum des Behälters 3 und der Innenseite der Membran 7.

In der Entnahmelage E wird die Membran 7 derart stark in Richtung Verschlusselement gekrümmt, dass die Federvorspannung die Kraftrichtung ändert und das Penetrationselement in der Entnahmelage fixiert. Die Reibung zwischen der Hohlnadel 9 und dem Verschlusselement 4 trägt weiter dazu bei, dass das Penetrationselement in dieser Position verbleibt.

Die eigentliche Entnahme der Flüssigkeit durch Aufziehen der Spritze 25 erfolgt selbstverständlich nicht in der dargestellten Position, sondern kopfüber, so dass die Flüssigkeit die Innenseite des Verschlusselements 4 flutet. Durch Aufziehen der Spritze fliesst so die Flüssigkeit in Pfeilrichtung f durch die Hohlnadel 9 in die Spritze 25. Der dabei entstehende Unterdruck über dem Flüssigkeitsspiegel wird ausgeglichen durch aus der Atmosphäre angesaugte Luft, welche in Pfeilrichtung 1 über die Ansaugöffnungen 23, durch das Filter 11 und über die Belüftungskanäle 10 in den Behälter 3 gelangt. Eine Kontamination der Flüssigkeit ist dabei nicht möglich, weil Schmutzteile oder beispielsweise auch Bakterien am Filter 11 ausgefiltert werden.

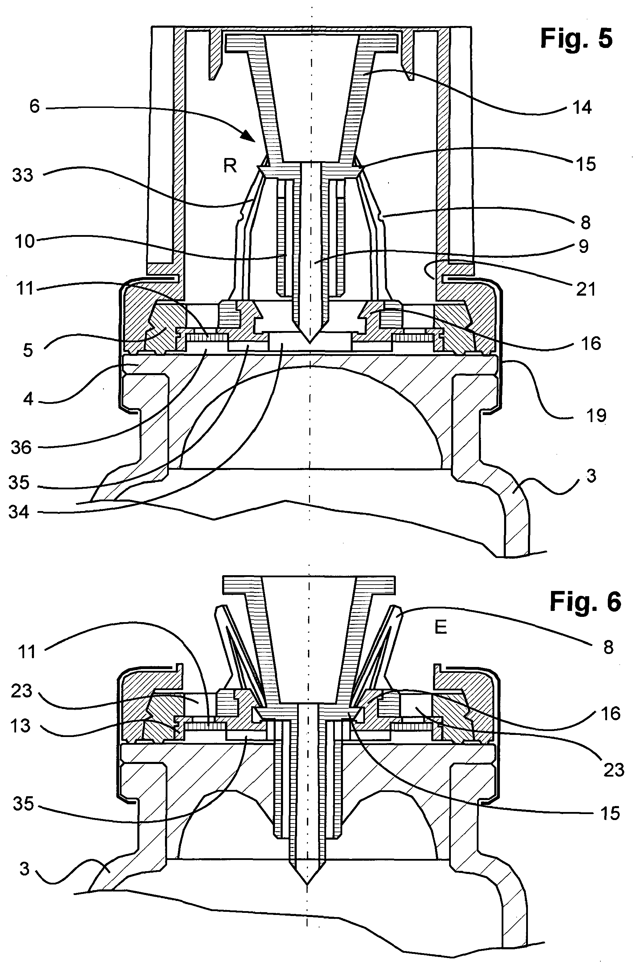

In den Figuren 5 und 6 ist ein alternatives Ausführungsbeispiel einer Vorrichtung dargestellt, wobei gleiche Bauteile mit den gleichen Bezugszeichen versehen sind, wie beim Ausführungsbeispiel gemäss den Figuren 1 und 2. Der wesentliche Unterschied besteht einerseits in der Verbindung zwischen dem Aufsatz 5 und dem Penetrationselement 6 und anderseits in der Anordnung des Filters 11. Auch die Verschlusskappe 18 ist etwas anders aufgebaut und nicht mit einem separaten Garantieband versehen. Vielmehr befindet sich die Sollbruchlinie 24 unmittelbar über dem eingegossenen oberen Rand der Manschette 19.

Der Aufsatz 5 ist mit dem Penetrationselement 6 über vier Materialstege 8 verbunden, welche um die Hohlnadel 9 gleichmässig verteilt sind. Jeder Materialsteg verfügt über wenigstens ein Biegegelenk 33. Die Materialstege 8 bilden auf diese Weise spinnenbeinartige Stützen, welche das Penetrationselement 6 in der Ruhelage halten. Der Luer-Lock 14 ist am Boden der Verschlusskappe 18 zusätzlich fixiert.

Beim vorliegenden Ausführungsbeispiel ist das Filter 11 nicht am Penetrationselement, sondern am Aufsatz 5 fixiert. Dazu ist eine separate Filterhalterung 13 vorgesehen, welche eine zentrale Öffnung aufweist und welche die Hohlnadel 9 kreisringförmig umgibt. Die innere, nabenartige Partie der Filterhalterung liegt auf dem Verschlusselement 4 auf, wobei jedoch die zentrale Öffnung 34 über Nuten 35 mit dem kreisringförmigen Hohlraum 36 verbunden ist, in dem das Filter 11 fixiert ist. An der Filterhalterung 13 ist ein nach innen ragender Rastring 16 angeordnet. Ebenso ist am Penetrationselement 6 ein nach aussen ragender Rastring 15 angeordnet.

In der Entnahmelage gemäss Figur 6 sind die Materialstege 8 um die Biegegelenke 33 gefaltet und ragen dabei etwas nach aussen. Die Rastringe oder Rastnasen 15 und 16 sind miteinander verrastet und fixieren auf diese Weise das Penetrationselement, dessen Hohlnadel 9 das Verschlusselement 4 durchstossen hat. Luft aus der Atmosphäre gelangt durch das Filter 11 über die Nuten 35 und die Belüftungskanäle 10 ins Innere des Behälters 3.

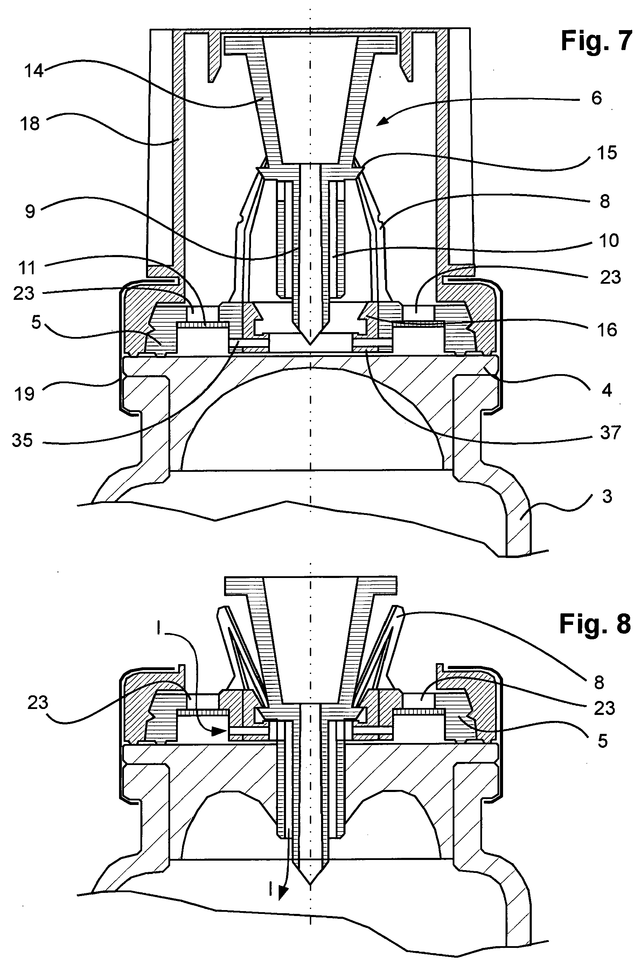

Das Ausführungsbeispiel gemäss den Figuren 7 und 8 unterscheidet sich von demjenigen gemäss den Figuren 5 und 6 nur durch die Art und Weise, wie das Filter 11 fixiert ist. Die Fixierung des Filters erfolgt hier nicht durch eine separate Filterhalterung, sondern unmittelbar durch einen Abschnitt des Aufsatzes 5. Separat ausgebildet ist hier lediglich ein Innenring 37, der den nach innen ragenden Rastring 16 trägt.

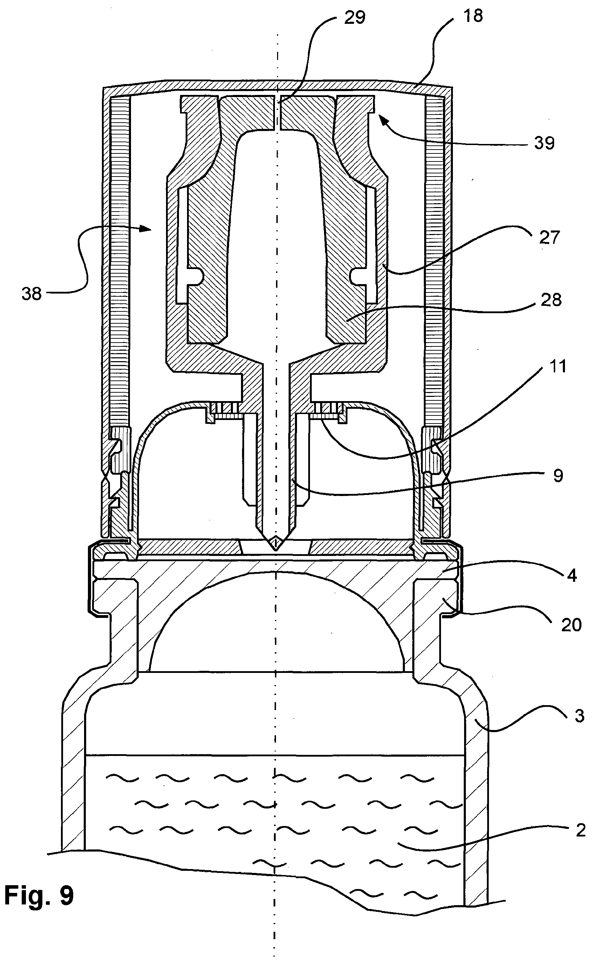

Das Ausführungsbeispiel gemäss Figur 9 entspricht weitgehend demjenigen gemäss Figur 1. In dem mit einem Ventilgehäuse 27 ausgebildeten, speziellen Kupplungsteil 39 ist ein mit 38 bezeichnetes Ventil eingebaut. Dieses Ventil ermöglicht es, in der Entnahmelage in mehreren zeitlich voneinander getrennten Sequenzen Flüssigkeit aus dem Behälter 3 zu entnehmen, wobei nach jeder Entnahme eine sterile Abdichtung gewährleistet ist. Im Ventilgehäuse 27 ist ein Ventilkörper 28 aus einem weichelastischen Material gelagert. In diesem Ventilkörper ist ein Schlitz 29 angeordnet, der im Ruhezustand geschlossen ist, so dass der Ventilkörper 28 wie eine Dichtung wirkt. Beim Ansetzen einer Spritze in den Luer-Lock 39 wird der Schlitz 29 aufgespreizt und gibt so den Zugang zur Hohlnadel 9 frei. Ein derartiges Ventil ist beispielsweise beschrieben in der WO 03/064907. Bedingt durch die grössere Bauhöhe des Penetrationselements 6 muss ersichtlicherweise auch die Verschlusskappe 18 länger ausgebildet werden.

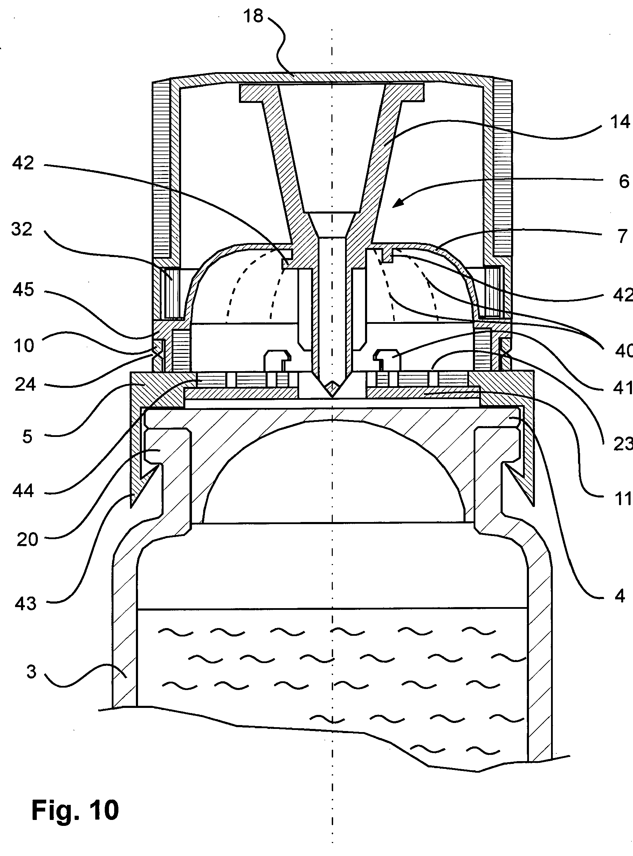

Das Ausführungsbeispiel gemäss Figur 10 hat zwar eine gewisse Ähnlichkeit mit demjenigen gemäss Figur 1, unterscheidet sich jedoch von diesem durch die Ausbildung der Membran 7, durch die Anordnung des Filters 11, sowie durch die Art der Befestigung auf der Behältermündung 20. Ausserdem wird das Penetrationselement 6 in der Entnahmelage durch eine Rastverbindung fixiert.

Die domartig gewölbte Membran 7 ist über den Umfang verteilt mit Sollbruchstellen oder Sollbruchlinien 40 versehen, welche sich etwa parallel zur Längsmittelachse vom Zenit der Kuppel bis zu deren unteren Rand erstrecken. Diese Sollbruchstellen können dabei als Schwächungslinien ausgebildet sein, oder es kann sich auch um Perforationen handeln. Beim Überführen des Penetrationselements aus der dargestellten Ruhelage in die Entnahmelage (analog zu Figur 2) platzen die Sollbruchstellen 40 auf, wobei schlitzartige Öffnungen entstehen. In der Entnahmelage rasten am Penetrationselement angeordnete Rastblöcke 42 in die Rasthaken 41 am Aufsatz 5 und fixieren auf diese Weise die Entnahmelage. Die Rastblöcke können ganz unterschiedlich ausgestaltet sein und beispielsweise wie in der linken Bildhälfte dargestellt, starr am Penetrationselement angeordnet sein. Denkbar ist aber auch eine Anordnung an der Membran 7, wie in der rechten Bildhälfte dargestellt (42'), wobei jeder Rastblock bis zum Erreichen der korrespondierenden Rasthaken 41 eine Schwenkbewegung von ca. 90° ausführt.

Der Aufsatz 5 ist hier nicht mittels einer Manschette an der Behältermündung fixiert, sondern mit Hilfe eines Schnappkragens 43 auf diese aufgeschnappt. Dieser Schnappkragen ist derart dimensioniert, dass einerseits eine dichtende Presskraft auf das Verschlusselement 4 ausgeübt wird und dass anderseits eine Entfernung der Schutzkappe 18 möglich ist, ohne dass unbeabsichtigt der Aufsatz 5 vom Behälter 3 abgehoben wird.

Das Filter 11 ist hier dem Aufsatz 5 zugeordnet, wobei es direkt unterhalb einer mit Ansaugöffnungen 23 versehenen Trägerplatte 44 befestigt ist. Die nachströmende Luft fliesst auf diese Weise über die geöffneten Sollbruchstellen 40, durch die Ansaugöffnungen 23 und das Filter 11 und danach über die Belüftungskanäle 10 ins innere des Behälters 3.

Beim vorliegenden Ausführungsbeispiel ist auch die Schutzkappe 18 mit der Sollbruchlinie 24 etwas abgewandelt ausgestaltet. Die domartige Membran 7 geht an ihrem unteren Rand in einen umlaufenden Stützring 45 über, der beispielsweise durch eine Schweissung einstückig mit dem Aufsatz 5 verbunden ist. Auf diesem Stützring ist eine Dichtung 32 abgestützt, welche die Innenseite der Schutzkappe 18 nach aussen hermetisch abdichtet. Der Dichtring 32 könnte auch als Zweikomponenten-Spritzgussteil direkt in die Schutzkappe 18 integriert sein. Der Materialstreifen unterhalb der Sollbruchlinie 24 ist als Garantieband ausgestaltet, nach dessen Entfernung die Schutzkappe 18 vom Stützring 45 abgehoben werden kann.

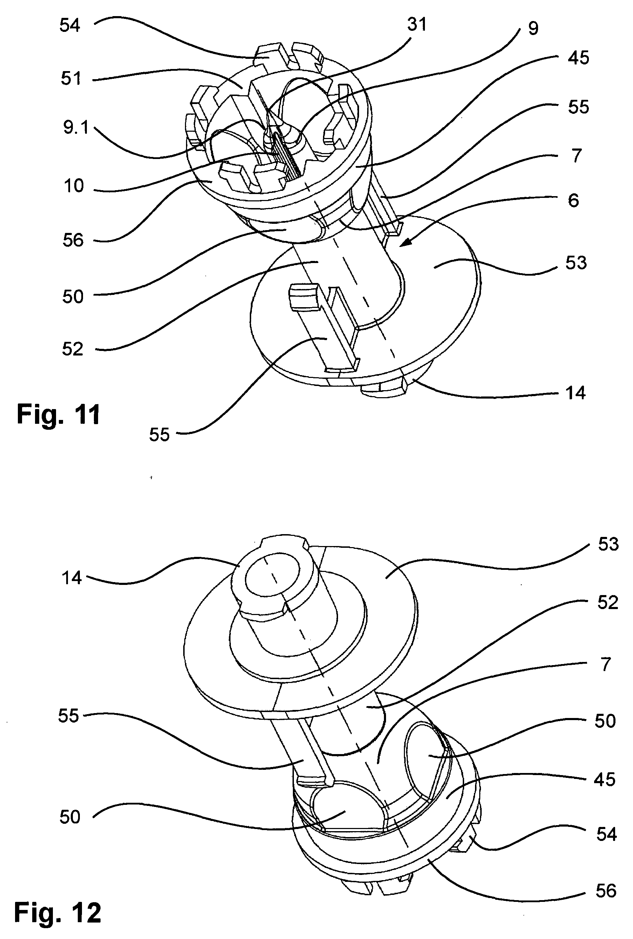

Figuren 11 bis 14 zeigen verschiedene Ansichten eines alternativen Ausführungsbeispiels eines Penetrationselements 6 einer erfindungsgemässen Vorrichtung und sind im Folgenden gemeinsam beschrieben. Dabei sind gleiche Bauteile mit gleichen Bezugszeichen versehen, wie bei den Ausführungsbeispielen gemäss den vorhergehenden Figuren. Das Ausführungsbeispiel der Fig. 11 und 12 (sowie auch 13 und 14) entspricht in weiten Teilen der in Fig. 10 dargestellten Vorrichtung. Ein wesentlicher Unterschied besteht einerseits in der Ausbildung der Membran 7, welche zur Verbindung des Penetrationselements 6 mit dem Aufsatz 5 vorgesehen ist, und andererseits in der Ausbildung von Rastelementen zur Verrastung des Penetrationselements 6 am Aufsatz 5 in der Entnahmelage.

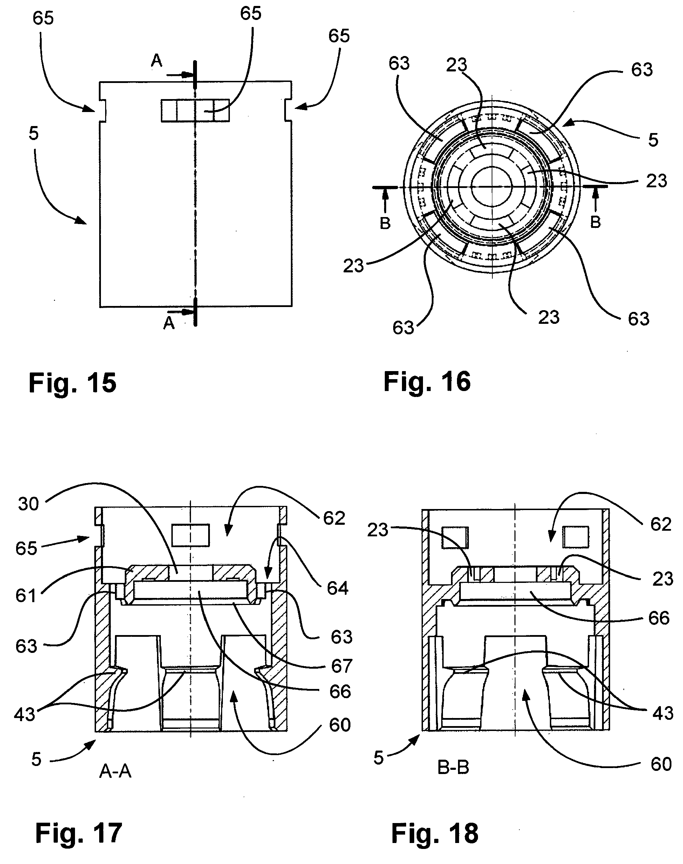

Die Membran 7 ist ebenfalls kuppelartig ausgebildet und geht randseitig über einen Stützring 45 in einen Befestigungsring 56 über, welcher zur Befestigung der Membran 7 am Aufsatz 5 vorgesehen ist. Hierzu weist der Stützring 45 vier stirnseitig angeordnete Rasthakenpaare 54 auf, welche zur Verrastung in entsprechenden Ausnehmungen 63 des Aufsatzes 5 vorgesehen sind (siehe Fig. 15-18)

Die Membran 7 weist vier im Wesentlichen kreisförmige Abflachungen 50 auf, welche z.B. von einer Sollbruchstelle begrenzt sein können. Innenwandig weist die Membran 7 vier Perforationsstege 51 auf, welche zwischen den kreisförmigen Abflachungen 50 im Wesentlichen in Längsrichtung angeordnet sind. Die Perforationsstege 51 sind steif ausgebildet und starr mit dem Stützring 45 sowie dem Befestigungsring 56 verbunden. Beim Übergang des Penetrationselements 6 in die Entnahmelage E zerreisen bzw. perforieren die Perforationsstege 51 die Membran 7. Damit ist sichergestellt, dass den Belüftungskanälen 10 der Hohlnadel 9 des Penetrationselements 6 Aussenluft zugeführt werden kann.

An die Membran 7 schliesst ein im Wesentlichen kreiszylindrischer Hals 52 des Penetrationselements 6 an, welcher sich ausserhalb der Membran 7 in Längsrichtung erstreckt. Endseitig geht der Hals 52 in das Luer-Lock 14 über. Zwischen Hals 52 und Luer-Lock 14 kragt flanschartig eine Fingerplatte 53 quer zur Längsrichtung des Penetrationselements 6 aus. Eine Fläche der Fingerplatte 53 ist dabei grösser ausgebildet, als ein Grundriss der Membran 7, so dass in einer Draufsicht in Längsrichtung die gesamte Membran 7 mit Stützring 45 und Befestigungsring 56 von der Fingerplatte 53 überdeckt ist.

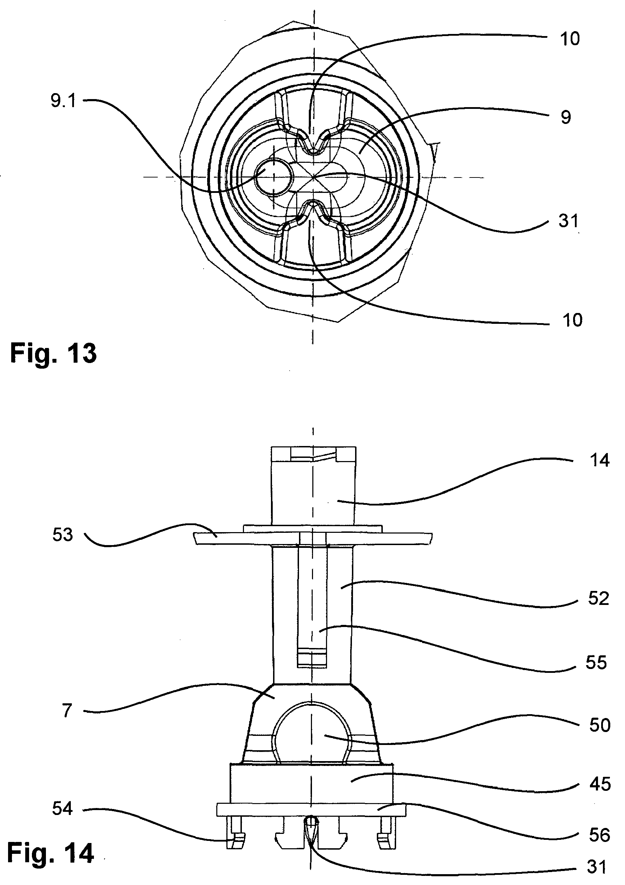

Das Luer-Lock 14 ist über einen Fluidkanal im Hals 52 mit einem Entnahmekanal 9.1 der Hohlnadel 9 des Penetrationselements 6 verbunden, welche zentral innerhalb der Membran 7 angeordnet ist. Die Hohlnadel 9 weist dabei zwei Belüftungskanäle 10 in der Form von V-förmigen Längsnuten auf, welche bezüglich der Längsachse gegenüberliegend an der Aussenseite der Hohlnadel 9 angeordnet sind. Der Entnahmekanal 9.1 der Hohlnadel 9 ist dabei exzentrisch angeordnet, sodass für eine gute Penetrationsfähigkeit eine Aufstechspitze 31 massiv ausgebildet sein kann (siehe auch Fig. 13)

An der Fingerplatte 53, in Längsrichtung zur Membran 7 sich erstreckend, sind zwei bezüglich der Längsrichtung gegenüberliegend angeordnete Rastzungen 55 ausgebildet. Die Rastzungen 55 dienen dabei der Verrastung des Penetrationselements 6 in der Entnahmelage E in entsprechenden Ausnehmungen 65 am Aufsatz 5 (siehe Fig. 15 bis 18). Die Rastzungen 55 (mit zugehörigen Ausnehmungen am Aufsatz 5) erfüllen damit eine analoge Funktion wie z.B. die Rastblöcke 42 bzw. 42' und Rasthaken 41 der Fig. 10. Im Unterschied dazu sind die Rastzungen 55 allerdings ausserhalb der Membran 7 angeordnet. Dies hat unter anderem den Vorteil, dass eine ordnungsgemässe Verrastung des Penetrationselements 6 am Aufsatz 5 auch von aussen überwacht werden kann bzw., sollte z.B. ein Eingriff nicht ordnungsgemäss erfolgen, der Eingriff manuell hergestellt werden kann.

Bevorzugt ist das Penetrationselement 6 zusammen mit der Membran und den übrigen in den Fig. 11 bis 14 dargestellten Elementen als einstückiger Formteil aus Kunststoff gespritzt. Selbstverständlich sind auch mehrteilige Ausführungen denkbar.

Figuren 15 bis 18 zeigen einen für das Penetrationselement 6 der Figuren 11 - 14 vorgesehenen Aufsatz 5 und sind im Folgenden gemeinsam beschrieben. Dabei sind gleiche Bauteile mit gleichen Bezugszeichen versehen, wie bei den Ausführungsbeispielen gemäss den vorhergehenden Figuren.

Der Aufsatz 5 hat eine weitgehend kreiszylindrische Form mit einem Aufnahmebereich 60 zur Anordnung der Behältermündung 20 des Behälters 3. Im Aufnahmebereich 60 ist innenwandig der Schnappkragen 43 ausgebildet, welcher vier nach innen vorstehende Rastvorsprünge aufweist. Der Aufnahmebereich 60 ist in Längsrichtung über eine quer zur Längsrichtung des Aufsatzes 5 angeordnete Zwischenwand 61 gegen einen Befestigungsbereich 62 für das Penetrationselement 6 abgegrenzt. Die Zwischenwand 61 weist eine zentrale Öffnung 30 auf, welche für den Durchtritt und zur Führung der Hohlnadel 9 des Penetrationselements 6 dient, wenn dieses über die Membran 7 am Aufsatz 5 befestigt ist und in die Entnahmelage E gebracht wird.

Die Zwischenwand 61 weist zudem Rastausnehmungen 63 für die Rasthakenpaare 54 der Membran 7 auf. Die Ausnehmungen 63 sind dabei kreisringförmig in einer kreisförmigen Aufnahmevertiefung 64 für den Befestigungsring 56 der Membran 7 angeordnet.

Im Befestigungsbereich 62 sind mantelseitig in radialer Richtung nach aussen offene Rastausnehmungen 65 ausgebildet, welche zum Eingriff der Rastzungen 55 des Penetrationselements 6 vorgesehen sind, wenn sich dieses in der Entnahmelage E befindet. Indem vier Rastausnehmungen 65 vorhanden sind, kann das Penetrationselement 6 in unterschiedlichen Ausrichtungen in den Aufsatz 5 eingesetzt werden, wobei eine Verrastung der Rastzungen 55 in jeder Ausrichtung sichergestellt ist.

Aufnahmeraumseitig ist an der Zwischenwand 61 ein Aufnahmebereich 66 für das Filter 11 ausgebildet. Ein umlaufender Dichtungsrand 67 des Aufnahmebereichs 66 ist derart ausgebildet, dass er dichtend auf dem Verschlusselement 4 aufliegt, wenn der Aufsatz 5 auf den Behälter 3 aufgesetzt ist. Im Bereich des Aufnahmebereichs 66 sind in der Zwischenwand 61 die Ansaugöffnungen 23 ausgebildet, welche den Befestigungsbereich 62 mit dem Aufnahmebereich 60 verbinden. Ist das Filter 11 im Aufnahmebereich 66 angeordnet, kann Aussenluft durch die Ansaugöffnungen 23 über das Filter 11 angesaugt und durch die Belüftungskanäle 10 der Hohlnadel 9 dem Innenraum des Behälter 3 zugeführt werden.

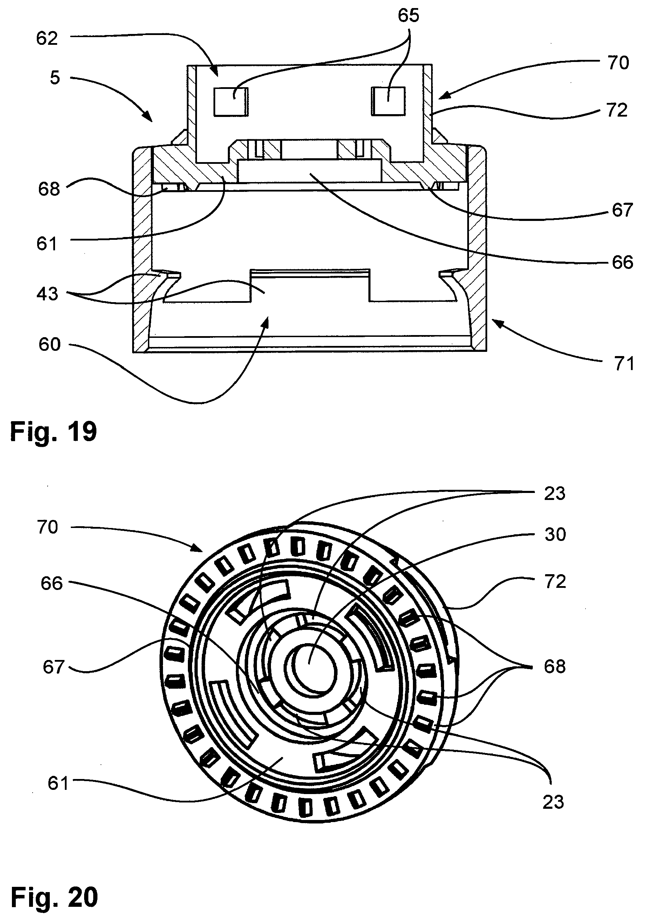

Figuren 19 und 20 zeigen eine weitere Ausführungsform des für das Penetrationselement 6 der Figuren 11 bis 14 vorgesehenen Aufsatzes 5 und sind im Folgenden gemeinsam beschrieben. Dabei sind gleiche Bauteile mit gleichen Bezugszeichen versehen, wie bei den Ausführungsbeispielen gemäss den vorhergehenden Figuren.

Im Gegensatz zur Ausführungsform der Fig. 15 bis 18 ist der Aufsatz 5 der Fig. 19 bis 20 zweiteilig ausgebildet, wobei ein rohrförmiges Anschlusssteil 71 eine weitgehend kreiszylindrische Form aufweist. Ein Innenraum des Anschlussteils 71 bildet den Aufnahmebereich 60 zur Anordnung der Behältermündung 20 des Behälters 3. Im Aufnahmebereich 60 ist innenwandig analog der Ausführungen der Fig. 15 bis 18 der Schnappkragen 43 ausgebildet, welcher vier nach innen vorstehende Rastvorsprünge umfasst.

Das Anschlussteil 71 ist einseitig von einem Kopplungsteil 70 verschlossen, welches die quer zur Längsrichtung des Aufsatzes 5 angeordnete Zwischenwand 61 umfasst. Das Kopplungsteil 70 weist eine in Längsrichtung nach aussen ragende kreiszylindrische Wand 72 auf, welche den Befestigungsbereich 62 für das Penetrationselement 6 bzw. die Membran 7 umschliesst.

Das Kopplungsteil 70 ist über die Zwischenwand 61 mit dem Anschlussteil 71 verbunden, welche das Anschlussteil 71 nach Art eines Deckels verschliesst. Die Zwischenwand 61 kann dabei form- , kraft- und/oder stoffschlüssig mit dem Anschlussteil 71 verbunden sein. Die Zwischenwand 61 weist analog der Ausführung der Fig. 15 bis 18 die Rastausnehmungen 63 für die Rasthakenpaare 54 der Membran 7 sowie die zentrale Öffnung 30 auf, welche für den Durchtritt und zur Führung der Hohlnadel 9 des Penetrationselements 6 dient.

Die Rastausnehmungen 65, welche zum Eingriff der Rastzungen 55 des Penetrationselements 6 vorgesehen sind, sind in der Ausführung der Fig. 19 und 20 in der kreiszylindrischen Wand 72 des Kopplungsteils 70 ausgebildet. Aufnahmeraumseitig ist an der Zwischenwand 61 der Aufnahmebereich 66 für das Filter 11 ausgebildet. Im Gegensatz zur Ausführung der Fig. 15 bis 18 ist der Dichtungsrand 67 von einem Rand der Ausnehmung 66 radial nach aussen beabstandet. Zusätzlich weist die Zwischenwand 61 aufnahmeraumseitig eine Vielzahl von strahlenförmig angeordneten Rastkeilen 68 auf, welche durch Eingriff in das z.B. elastisch ausgebildete Verschlusselement 4 ein Verdrehen des Behälters 3 gegenüber der Rastplatte 61 verhindern. Es versteht sich, dass derartige Rastkeile auch bei sämtlichen anderen Ausführungsformen eines Aufsatzes vorgesehen sein können. Der Aufsatz der Fig. 19 und 20 ist insbesondere für den Anschluss an Behälter mit einem 20mm Bördelrand vorgesehen.

Für sämtliche erfindungsgemässen Ausführungen können als Filter neben den gängigen Mikrofiltermembranen vorteilhaft beispielsweise PET-Folien oder Polykarbonat-Folien verwendet werden, welche von feinsten Öffnungen durchsetzt sind. Anstelle von separat eingelegten oder angeklebten Filtern könnten diese auch direkt beim Spritzgiessen der jeweiligen Tragkonstruktionen integriert werden. Ein wesentlicher Vorteil sämtlicher erfindungsgemässen Varianten besteht auch noch darin, dass diese für Behälter mit einem Bördelrand von 13 mm oder 20 mm Durchmesser bzw. für Behälter mit Bördelrändern beliebigen Durchmessers verwendet werden können. Insbesondere versteht sich, dass jede Ausführung entsprechend angepasst werden kann.

## Patentansprüche

1. Vorrichtung (1) für die Entnahme einer Flüssigkeit (2) aus einem Behälter (3), der mit einem penetrierbaren Verschlusselement (4) verschlossen ist, mit einem dichtend an das Verschlusselement anschliessbaren Aufsatz (5), in welchem ein Penetrationselement (6) derart gelagert ist, dass es mit Hilfe von Führungsmitteln (7, 8) zwischen einer Ruhelage (R) und einer Entnahmelage (E) verschiebbar ist, wobei das Penetrationselement wenigstens eine Hohlnadel (9) aufweist, mit welcher in der Entnahmelage das Verschlusselement penetrierbar ist, mit wenigstens einem Belüftungskanal (10) zum Belüften des Behälters (3) bei der Entnahme der Flüssigkeit, wobei die Führungsmittel (7, 8) wenigstens einen Wandabschnitt aufweisen, der das Penetrationselement (6) mit dem Aufsatz (5) verbindet und dieses in der Ruhelage (R) hält, wobei der Wandabschnitt derart verformbar ist, dass er das Penetrationselement bis zum Erreichen der Entnahmelage (E) führt, **dadurch gekennzeichnet, dass** der Wandabschnitt wenigstens eine das Penetrationselement umgebende, federelastische Membran (7) ist.

2. Vorrichtung nach Anspruch 1, **dadurch gekennzeichnet, dass** der Belüftungskanal als Nut auf der Aussenseite der Hohlnadel ausgebildet ist.

3. Vorrichtung nach Anspruch 1 oder 2, **dadurch gekennzeichnet, dass** sie wenigstens ein Filter (11) aufweist, über das in der Entnahmelage Luft aus der Atmosphäre über den Belüftungskanal (10) in den Behälter zuführbar ist.

4. Vorrichtung nach Anspruch 3, **dadurch gekennzeichnet, dass** das Filter am Penetrationselement (6), vorzugsweise am atmosphärenseitigen Ende des Belüftungskanals angeordnet ist.

5. Vorrichtung nach Anspruch 4, **dadurch gekennzeichnet, dass** am atmosphärenseitigen Ende des Belüftungskanals (10) ein die Hohlnadel (9) umgebender Kragen (12) angeordnet ist und dass das Filter am Kragen gehalten ist.

6. Vorrichtung nach einem der Ansprüche 3 bis 5, **dadurch gekennzeichnet, dass** das Filter (11) am Aufsatz (5), vorzugsweise in einem dem Verschlusselement zugewandten Bereich angeordnet ist.

7. Vorrichtung nach einem der Ansprüche 1 bis 6, **dadurch gekennzeichnet, dass** die Führungsmittel (7, 8) einstückig mit dem Aufsatz (5) und/oder mit dem Penetrationselement (6) verbunden sind.

8. Vorrichtung nach einem der Ansprüche 1 bis 6, **dadurch gekennzeichnet, dass** die Führungsmittel (7, 8) Befestigungsmittel aufweisen, welche zum Verbinden der Führungsmittel (7, 8) mit dem Aufsatz (5), bevorzugt unlösbar, am Aufsatz (5) verrastbar sind.

9. Vorrichtung nach einem der Ansprüche 1 bis 8, **dadurch gekennzeichnet, dass** der Aufsatz (5) zweiteilig oder mehrteilig ausgebildet ist, wobei ein erster Teil zum Ankoppeln an den Behälter (3) vorgesehen ist und ein zweiter Teil mit dem Penetrationselement verbunden ist.

10. Vorrichtung nach einem der Ansprüche 1 bis 9, **dadurch gekennzeichnet, dass** das Penetrationselement (6) ein Kupplungsteil, insbesondere den Innenkegel eines Luer-Locks aufweist.

11. Vorrichtung nach einem der Ansprüche 1 bis 10, **dadurch gekennzeichnet, dass** das Penetrationselement (6) Rastmittel aufweist, mit denen es in der Entnahmelage (E) verrastbar ist.

12. Vorrichtung nach Anspruch 11, **dadurch gekennzeichnet, dass** die Rastmittel derart am Penetrationselement (6) angeordnet sind, dass sie zur Manipulation durch einen Benutzer von aussen zugänglich sind, wobei das Penetrationselement (6) vorzugsweise eine Fingerplatte zum Verschieben zwischen Ruhelage (R) und Entnahmelage (E) aufweist, an welcher die Rastmittel ausgebildet sind.

13. Vorrichtung nach Anspruch 11 oder 12, **dadurch gekennzeichnet, dass** der Aufsatz Rastmittel aufweist, welche zu den Rastmitteln des Penetrationselements (6) komplementär sind, sodass das Penetrationselement (6) mit seinen Rastmitteln am Aufsatz (5) verrastbar ist.

14. Vorrichtung nach einem der Ansprüche 1 bis 13, **dadurch gekennzeichnet, dass** der Aufsatz (5) einen Zentrierring (17) zum Zentrieren der Hohlnadel trägt.

15. Vorrichtung nach einem der Ansprüche 1 bis 14, **dadurch gekennzeichnet, dass** das Penetrationselement (6) in der Ruhelage durch eine lösbare Schutzkappe (18) abgedeckt ist.

16. Vorrichtung nach einem der Ansprüche 1 bis 15, **dadurch gekennzeichnet, dass** am Penetrationselement (6) für die mehrfache Entnahme von Flüssigkeit in der Entnahmelage ein Ventil zum vorzugsweise sterilen Verschliessen der Hohlnadel angeordnet ist.

17. Anordnung mit einem eine Flüssigkeit enthaltenden Behälter, dessen Behältermündung mit einem penetrierbaren Verschlusselement (4) verschlossen ist, sowie mit einer Vorrichtung nach einem der Ansprüche 1 bis 16, wobei der Aufsatz (5) dichtend auf dem Verschlusselement aufliegt und insbesondere auf der Behältermündung (20) aufgeschnappt ist.

## Claims

1. Device (1) for withdrawing a liquid (2) from a container (3), which is sealed by a penetrable sealing element (4), with a head piece (5), which can be tightly attached to the sealing element, in which a penetration element (6) is mounted in such a way that it is displaceable with the aid of guide means (7, 8) between an idle position (R) and a withdrawal position (E), wherein the penetration element comprises at least one hollow needle (9), by means of which the sealing element is penetrable in the withdrawal position, having at least one aeration channel (10) for aerating the container (3) as the liquid is withdrawn, wherein the guide means (7, 8) comprise at least one wall portion, which connects the penetration element (6) to the head piece (5) and holds said penetration element in the idle position (R), wherein the wall portion is deformable in such a way that it guides the penetration element until the withdrawal position (E) is reached, **characterized in that** the wall portion is at least one resilient membrane (7) surrounding the penetration element.

2. Device according to Claim 1, **characterized in that** the aeration channel is formed as a groove on the outer face of the hollow needle.

3. Device according to Claim 1 or 2, **characterized in that** it comprises at least one filter (11), via which air from the atmosphere can be fed in the withdrawal position into the container via the aeration channel (10).

4. Device according to Claim 3, **characterized in that** the filter is arranged on the penetration element (6), preferably at the atmosphere-side end of the aeration channel.

5. Device according to Claim 4, **characterized in that** a collar (12) surrounding the hollow needle (9) is arranged at the atmosphere-side end of the aeration channel (10), and **in that** the filter is held on the collar.

6. Device according to one of Claims 3 to 5, **characterized in that** the filter (11) is arranged on the head piece (5), preferably in a region facing towards the sealing element.

7. Device according to one of Claims 1 to 6, **characterized in that** the guide means (7, 8) are connected integrally to the head piece (5) and/or to the penetration element (6).

8. Device according to one of Claims 1 to 6, **characterized in that** the guide means (7, 8) comprise fastening means, which, in order to connect the guide means (7, 8) to the head piece (5), can be latched, preferably non-releasably, to the head piece (5).

9. Device according to one of Claims 1 to 8, **characterized in that** the head piece (5) is formed in two or more parts, wherein a first part is provided to be coupled to the container (3) and a second part is connected to the penetration element.

10. Device according to one of Claims 1 to 9, **characterized in that** the penetration element (6) comprises a coupling part, in particular the female taper of a Luer lock.

11. Device according to one of Claims 1 to 10, **characterized in that** the penetration element (6) comprises detent means, by means of which it can be latched in the withdrawal position (E).

12. Device according to Claim 11, **characterized in that** the detent means are arranged on the penetration element (6) in such a way that they are externally accessible for manipulation by a user, wherein the penetration element (6) preferably comprises a fingerplate for displacement between the idle position (R) and withdrawal position (E), the detent means being formed on said fingerplate.

13. Device according to Claim 11 or 12, **characterized in that** the head piece comprises detent means, which are complementary to the detent means of the penetration element (6) such that the penetration element (6) can be latched by means of its detent means to the head piece (5).

14. Device according to one of Claims 1 to 13, **characterized in that** the head piece (5) carries a centering ring (17) for centering the hollow needle.

15. Device according to one of Claims 1 to 14, **characterized in that** the penetration element (6) is covered in the idle position by a releasable protective cap (18).

16. Device according to one of Claims 1 to 15, **characterized in that**, for multiple withdrawals of liquid in the withdrawal position, a valve for sealing the hollow needle, preferably in a sterile manner, is arranged on the penetration element (6).

17. Arrangement comprising a container containing a liquid, the container mouth of said container being sealed by a penetrable sealing element (4), and also comprising a device according to one of Claims 1 to 16, wherein the head piece (5) rests tightly on the sealing element and in particular is snapped onto the container mouth (20).

## Revendications

1. Ensemble (1) permettant de prélever un liquide (2) dans un récipient (3) fermé par un élément de fermeture (4) perforable, l'ensemble présentant
une garniture (5) apte à être raccordée de manière hermétique à l'élément de fermeture, dans laquelle un élément de perforation (6) est monté de telle sorte qu'il puisse coulisser à l'aide de moyens de guidage (7, 8) entre une position de repos (R) et une position de prélèvement (E), l'élément de perforation présentant au moins une aiguille creuse (9) par laquelle il peut perforer l'élément de fermeture dans la position de prélèvement,
au moins un canal d'évent (10) qui introduit de l'air dans le récipient (3) lors du prélèvement du liquide, les moyens de guidage (7, 8) présentant au moins une section de paroi qui relie l'élément de perforation (6) à la garniture (5) et maintient cette dernière en position de repos (R), la section de paroi pouvant être déformée de telle sorte qu'elle guide l'élément de perforation jusqu'à ce qu'il atteigne la position de prélèvement (E),
**caractérisé en ce que**
la section de paroi est au moins une membrane élastique (7) qui entoure l'élément de perforation.

2. Ensemble selon la revendication 1, **caractérisé en ce que** le canal d'évent est configuré comme rainure ménagée sur le côté extérieur de l'aiguille creuse.

3. Ensemble selon les revendications 1 ou 2,
**caractérisé en ce qu'**il présente au moins un filtre (11) par l'intermédiaire duquel l'air provenant de l'atmosphère peut être amené dans le récipient par l'intermédiaire du canal d'évent (10) dans la position de prélèvement.

4. Ensemble selon la revendication 3, **caractérisé en ce que** le filtre est disposé sur l'élément de perforation (6), de préférence à l'extrémité du canal d'évent situé du côté de l'atmosphère.

5. Ensemble selon la revendication 4, **caractérisé en ce qu'**un collet (12) qui entoure l'aiguille creuse (9) est disposé à l'extrémité du canal d'évent (10) situé du côté de l'atmosphère et **en ce que** le filtre est maintenu sur le collet.

6. Ensemble selon l'une des revendications 3 à 5,
**caractérisé en ce que** le filtre (11) est disposé sur la garniture (5) et de préférence dans la partie de cette dernière qui est tournée vers l'élément de fermeture.

7. Ensemble selon l'une des revendications 1 à 6,
**caractérisé en ce que** les moyens de guidage (7, 8) se raccordent d'un seul tenant à la garniture (5) et/ou à l'élément de perforation (6).

8. Ensemble selon l'une des revendications 1 à 6,
**caractérisé en ce que** les moyens de guidage (7, 8) présentent des moyens de fixation qui peuvent être encliquetés sur la garniture (5), de préférence de manière non libérable, pour relier les moyens de guidage (7, 8) à la garniture (5).

9. Ensemble selon l'une des revendications 1 à 8,
**caractérisé en ce que** la garniture (5) est réalisée en deux pièces ou en plusieurs pièces, une première partie étant prévue pour le raccordement au récipient (3) et une deuxième partie étant reliée à l'élément de perforation.

10. Ensemble selon l'une des revendications 1 à 9,
**caractérisé en ce que** l'élément de perforation (6) présente une pièce d'accouplement, en particulier le cône intérieur d'un raccord Luer.

11. Ensemble selon l'une des revendications 1 à 10,
**caractérisé en ce que** l'élément de perforation (6) présente des moyens d'encliquetage qui permettent de l'encliqueter dans la position de prélèvement (E).

12. Ensemble selon la revendication 11, **caractérisé en ce que** les moyens d'encliquetage sont disposés sur l'élément de perforation (6) de telle sorte qu'ils soient accessibles de l'extérieur pour permettre leur manipulation par un utilisateur, l'élément de perforation (6) présentant de préférence une plaque de doigt destinée à coulisser entre la position de repos (R) et la position de prélèvement (E) et sur laquelle sont formés les moyens d'encliquetage.

13. Ensemble selon les revendications 11 ou 12,
**caractérisé en ce que** la garniture présente des moyens d'encliquetage qui sont complémentaires des moyens d'encliquetage de l'élément de perforation (6) de telle sorte que l'élément de perforation (6) peut être encliqueté sur la garniture (5) par ses moyens d'encliquetage.

14. Ensemble selon l'une des revendications 1 à 13,
**caractérisé en ce que** la garniture (5) porte un anneau de centrage (17) qui permet de centrer l'aiguille creuse.

15. Ensemble selon l'une des revendications 1 à 14,
**caractérisé en ce que** l'élément de perforation (6) est recouvert en position de repos par un capot de protection (18) libérable.

16. Ensemble selon l'une des revendications 1 à 15,
**caractérisé en ce qu'**une soupape de fermeture de préférence stérile de l'aiguille creuse est disposée sur l'élément de perforation (6) pour permettre plusieurs prélèvements de liquide dans la position de prélèvement.

17. Système présentant un récipient contenant un liquide et dont l'embouchure est fermée par un élément de fermeture (4) perforable, ainsi qu'un ensemble selon l'une des revendications 1 à 16, la garniture (5) reposant de manière hermétique sur l'élément de fermeture et en particulier étant fixée élastiquement sur l'embouchure (20) du récipient.
